# EUROPEAN PATENT SPECIFICATION

(11) **EP 2 373 254 B1**
(45) Date of publication and mention of the grant of the patent: **13.11.2013**
(21) Application number: 09799287.9
(22) Date of filing: 28.12.2009
(51) Int. Cl.: A61F 2/04

(54) **ORTHOTOPIC ARTIFICIAL BLADDER PROSTHESIS**
ORTHOTOPISCHE KÜNSTLICHE HARNBLASENPROTHESE
PROTHÈSE DE VESSIE ARTIFICIELLE ORTHOTOPIQUE

(30) Priority: 07.01.2009 IT MI20090004
(43) Date of publication of application: 12.10.2011
(73) Proprietor: Sambusseti, Antonio, 26100 Cremona (IT)
(72) Inventor: Sambusseti, Antonio, 26100 Cremona (IT)
(74) Representative: Petruzziello, Aldo
(86) International application number: PCT/EP2009/009280
(87) International publication number: WO 2010/078949

(56) References cited:
- WO-A-02/058594
- WO-A-03/028546
- WO-A-2007/039159
- WO-A-2007/039160
- FR-A- 2 116 838
- NL-A- 7 416 695

## Description

The present invention refers to an orthotopic artificial bladder prosthesis.

As is known, when a patient's bladder is affected by severe incurable disease which compromises its proper functioning, replacement of the bladder with an artificial bladder prosthesis is desirable.

Among the various solutions developed to solve the problem is replacement of the natural bladder with a compressible bladder made of soft multi-layer silicone emptying of which takes place by simply compressing the lower abdomen, as described in patent WO 2007/039159. This type of prosthesis provides a connection between the ureters and urethra and said artificial bladder through the use of sutures. However, securing with sutures is not always possible, as in the case, for example, of the ureters and/or urethra being weakened and/or thinned for congenital or pathological reasons. Furthermore, the use of sutures requires high manual skill as well as further use of time during the replacement procedure.

Object of the present invention is to overcome the drawbacks of the prior art, by providing an orthotopic artificial bladder prosthesis that is able to replace the natural bladder and which allows a considerable improvement in the patient's quality of life.

Another object of the present invention is to provide such an orthotopic artificial bladder prosthesis that does not require the use of sutures to secure the ureters and/or the urethra to said artificial bladder thus avoiding the risk of tissue necrosis.

Yet another object of the present invention is to provide such an orthotopic artificial bladder prosthesis that is easy to produce.

These objects are achieved in accordance with the invention with the characteristics listed in appended independent claim 1.

Advantageous embodiments of the invention will be apparent from the dependent claims.

The orthotopic artificial bladder prosthesis according to the invention comprises an enclosure or bag or balloon made from a multi-layer membrane of soft and elastic synthetic material, the inner layer of which is able not to deteriorate with urine whereas the outer layer is suitable to prevent fusing with the surrounding tissues. Said orthotopic prosthesis further comprises at least two holes for connecting the ureters to said prosthesis and the same number of hollow elements having a widened circular flat base and a tubular portion passing through said holes which has substantially the same inner diameter as the outer diameter of ureters such as to accomplish an interference fit without the necessity to use glues for fixing the ureters inside said tubular portion. The ureters are then inserted forcibly inside said tubular portion of the hollow element without substantially narrowing the ureters lumen.

Further characteristics of the invention will be made clearer by the detailed description that follows, referring to a purely exemplifying and therefore non limiting embodiment thereof, illustrated in the appended drawings, in which:
Figure 1 is a perspective view of an orthotopic artificial bladder prosthesis in which the ureter is shown connected to said prosthesis by means of a hollow element according to the present invention whereas the other ureter and urethra are connected to the prosthesis by means of sutures according to the prior art;
Figure 2 is an enlarged cross sectional view of a portion of the artificial bladder taken along the section II-II of Figure 1;
Figure 3 is an exploded, cut-away perspective view showing the assembly between the hollow element and a ureter;
Figure 4 is an enlarged sectional view at mid-length showing a ureter assembled in the hollow element mounted on the artificial prosthesis.
Figures 5a and 5b are, respectively, a top view and a mid-length sectional view of a second embodiment of the hollow element;
Figure 6 is an enlarged sectional view at mid-length showing the hollow element of

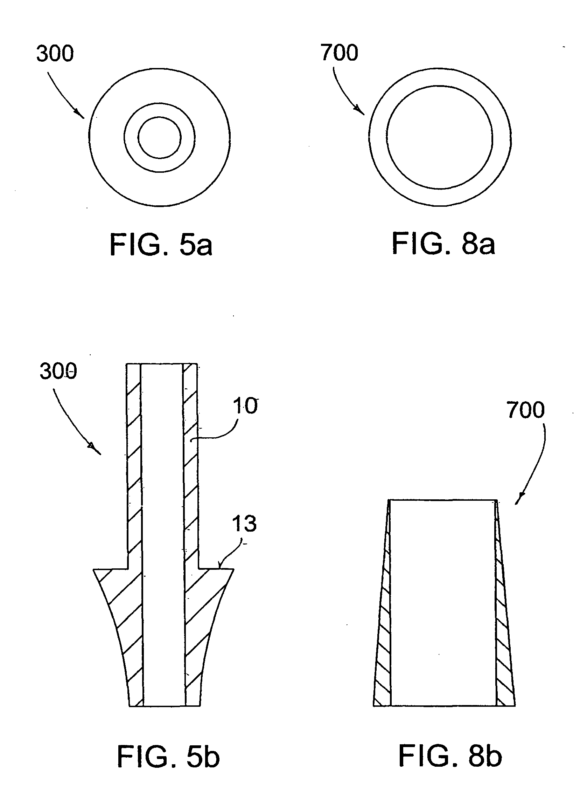

Figure 5a mounted on the artificial prosthesis in which a ureter is inserted;

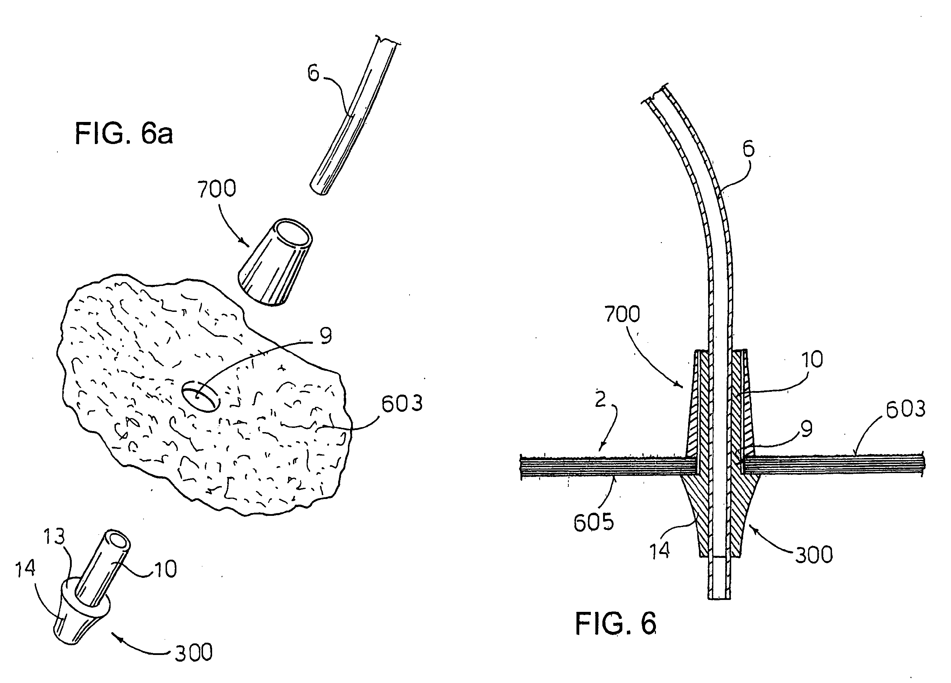

Figure 6a is a perspective exploded, cut-away view showing the assembly between the hollow element of Figure 5a and a ureter;

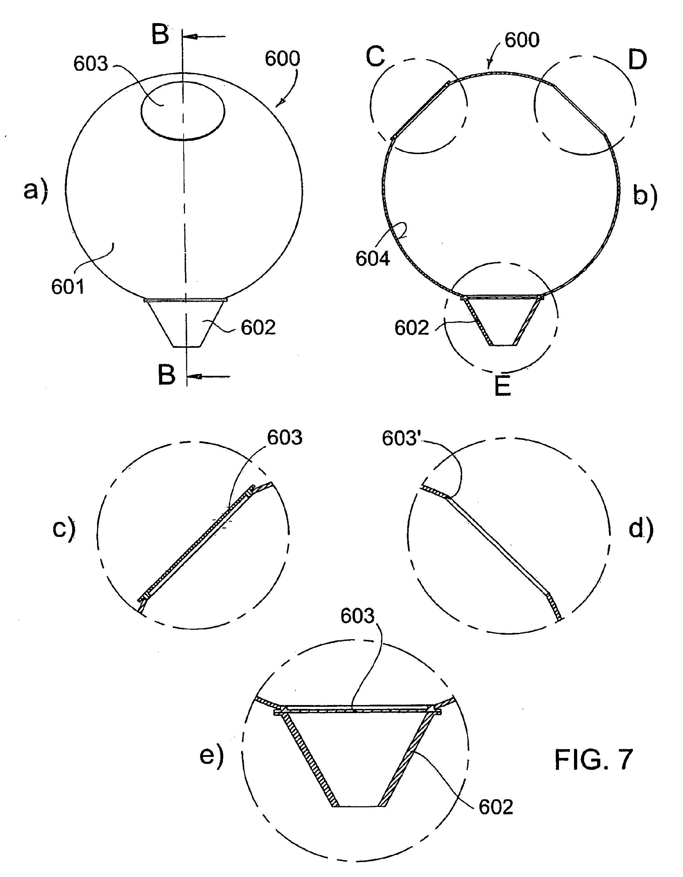

Figure 7a is a front view of a precursor of an orthotopic artificial bladder prosthesis of a second embodiment to be coupled to the hollow element illustrated in Figures 5a-5b;
Figure 7b is a sectional view of Figure 7a taken along the line B-B;
Figures 7c, 7d, 7e are sectional views of the details indicated respectively with C, D and E in Figure 7b;
Figures 8a e 8b are, respectively, a top view and a mid-length sectional view of a sleeve to be coupled to the hollow element of Figures 5a-5b.

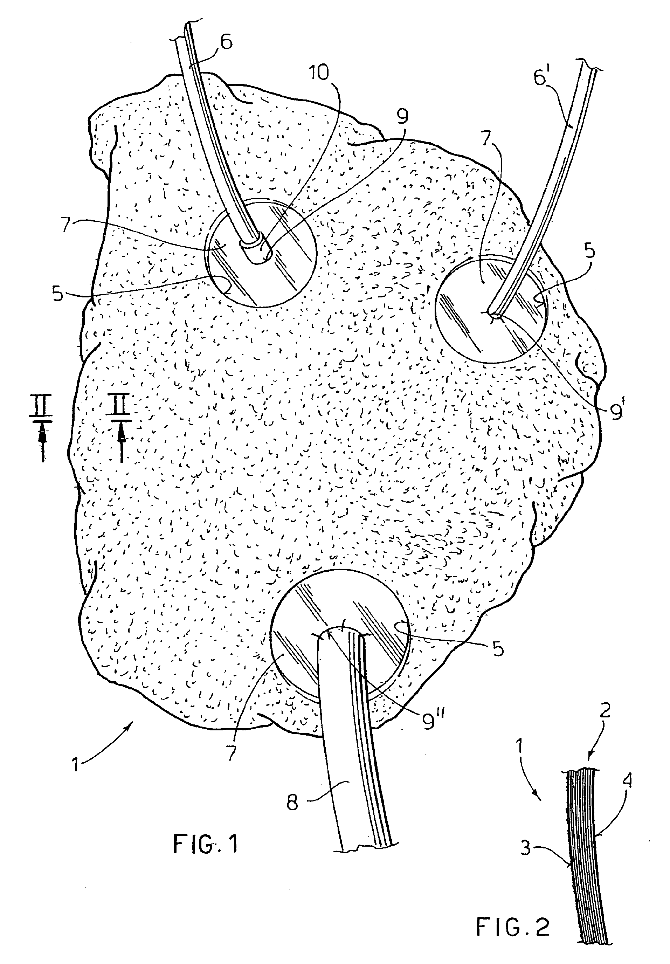

In Fig. 1 a bladder prosthesis, designated as a whole with reference numeral 1, in the form of an enclosure or bag or collapsible balloon is illustrated. That is to say, the mechanism for filling and emptying of the prosthesis 1 works through the effect of the differences in pressure between the air inside the prosthesis and the inner liquid (urine), both inside the prosthesis 1. The capacity of said prosthesis 1 is between 100 and 900 cm³.

The prosthesis I of Figure 1 consists of a multilayer membrane 2 (Figure 2) made of soft silicone, with a thickness so as to be compressible, deflatable or collapsible. The membrane of the prosthesis preferably has a thickness of about 600 microns and consists of 20 layers of silicone, each having a thickness of about 30 microns. The silicone used can consist, for example, of copolymers of dimethyl and metavinyl siloxane, reinforced with silicon. A medical silicone is preferably used, such as, for example, that known by the code number MED 4735™ and marketed by Nusil Technology.

The outermost layer 3 of the silicone membrane 2 is coated with turbostratic pyrolytic carbon, in order to reduce the risk of adhesion of the fibrous capsule to the prosthesis 1. The inner surface of the prosthesis 1, on the other hand, is coated with a microfilm 4 of highly biocompatible material, such as pyrolytic turbostratic carbon, for example, having a thickness of about 0.2-0.3 microns.

The process for obtaining said membrane is described in patent application WO 2007/039159.

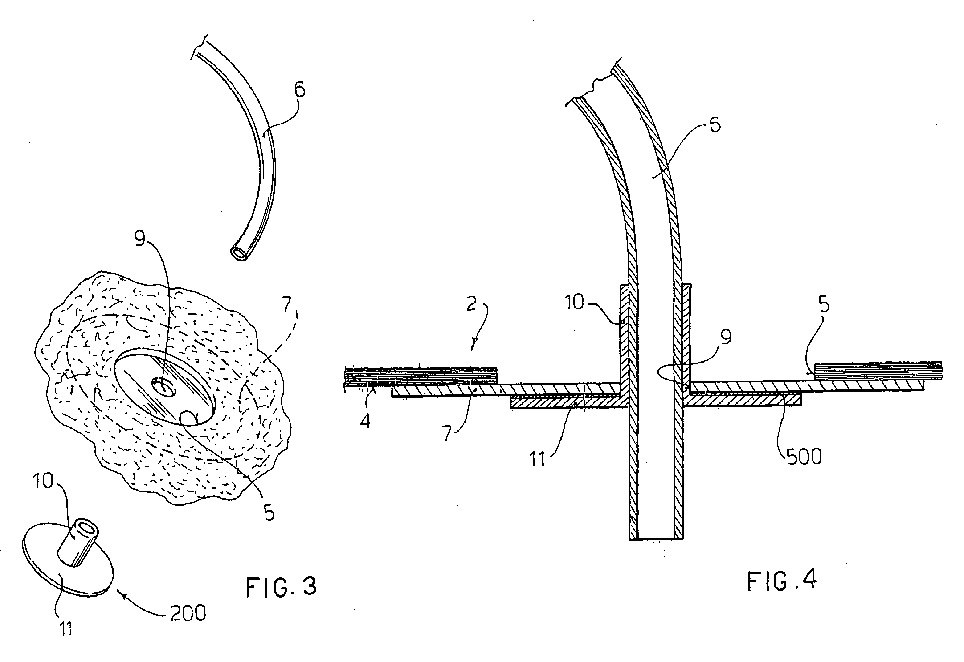

The prosthesis 1 shown in Figure 1 is also provided with three portions of membrane 7 which have a hole 9, 9', 9" centrally. Said portions of membrane 7 are applied to the inner surface 4 (Figure 4) of the membrane 2 of the prosthesis 1 to cover the holes 5 previously formed on said prosthesis 1 according to what is described in WO 2007/039159.

Said portions 7 of membrane, which are of a larger size than the hole 5 (Figure 4), are generally used in artificial bladders in order to facilitate suturing of the ureters and the urethra to the prosthesis 1. Said portions 7 of membrane can be similar to the multi-layer membrane 2 which forms the bag of the prosthesis 1 or without the texturized layer 3.

As shown in Figure 1, according to the invention, the ureter 6 is connected to the membrane 7 by means of a tubular portion 10 inside which said ureter 6 is inserted. The tubular portion 10 forms part of a hollow element shown in Figure 3 and designated with reference numeral 200. Said hollow element 200 also has a widened circular flat base 11 from which said tubular portion 10 begins. Said tubular portion 10 has the longitudinal axis perpendicular to said circular base 11 and has an inner diameter equal along its length as it has no inner recesses and/or projections. On the circular base 11 there is placed a silicone glue 500 (Figure 4) which allows the hollow element 200 to be made integral with the membrane 7, in turn integral with the membrane 2 of the prosthesis 1, after the tubular portion 10 of said hollow element 200 has been inserted in the hole 9 present on the membrane 7 as illustrated in Figure 4.

The tubular portion 10 has an inner diameter between 5 and 30 charrier (Ch) or between 5 and 15 Ch, a thickness between 1.0 and 10 mm and a length not exceeding about 5 cm. The circular base 11 has a diameter not exceeding that of the hole 5 on which the membrane 7 is applied.

The hole 9 on the membrane 7 generally has a size between 5 and 30 Ch unit and is such as to allow forcible passage of the tubular part 10 of the hollow element 200. It should be noted that 1 Ch corresponds to 1/3 mm.

The hollow element 200 inclusive of the tubular portion 10 is coated, both on the inside and on the outside, with pyrolytic turbostratic carbon.

During the surgical stage the ureter 6 is inserted forcibly inside the tubular portion 10 of the hollow element 200 which grips lightly around the tube of the ureter 6 thanks to its elasticity. The ureter is inserted inside the tubular portion 10 so as to protrude with respect to the circular base 11 of the hollow element 200 as illustrated in Figure 4.

In this manner it is no longer necessary to fix the ureter 6 to the portions of membrane 7 by means of sutures as happens, on the other hand, in the prior art illustrated in Figure 1 for the ureter 6'. That allows to avoid necrosis phenomena which can occur in case of sutures. In addition the interference fit between ureter and the hollow element 200 prevents to use and apply glues on ureter surface.

In another embodiment (not illustrated in the figure) of the invention the membrane 7 is absent and the hole 9 which allows the passage of the hollow element 200 is formed directly on the membrane 2 of the prosthesis 1 without having to make the hole 5 illustrated in Figure 1, and cover it by means of the membrane 7.

Naturally, the hollow element 200, suitably sized, can also be used to connect the ureter 8 to the prosthesis 1.

Furthermore, the artificial prosthesis 1 on which the hollow elements 200 are used to secure the ureters 6, 6' according to the present invention can be an artificial prosthesis which has a catheter or stent in place of the urethra 8.

As an alternative to the embodiment illustrated in Figure 4, the system represented by the tubular body 200 comprising the elements 10, 11 can be replaced by an element 300 illustrated in Figures 5 and 6.

Said element 300 is a single piece and has a tubular portion 10 in which to insert the ureter similarly to what was described previously in relation to element 200, and a truncated conical portion 14 connected to said tubular portion 10 by means of a circular surface 13 (Figure 5b) which has the function of allowing gluing of said element 300 to the inner surface of an artificial bladder 1. Said element 300 is made of silicone with a hardness of about 50 Shore.

In a preferred embodiment of said element 300, the tubular portion 10 has an inner diameter of about 6 mm and an outer diameter of about 10 mm, a total length of about 50 mm, of which 30 mm represent the length of the tubular part 10, and a diameter of the circular surface 13 of about 20 mm.

Said one-piece element 300 is coated with pyrolitic turbostratic carbon both internally and externally, and is used in combination with an artificial bladder also coated with said turbostratic carbon both internally and externally but not texturized.

Coupling of said single piece 300 as defined above to an artificial bladder or prosthesis 1, also coated with turbostratic carbon both internally and externally but not texturized, is achieved through a process having a sequence of stages which will be described hereunder.

To start with, a prosthesis in the form of a balloon 600 (Figure 7a) consisting of a membrane 2 made of soft silicone is obtained.

Said membrane 2 can be multi-layered and made with a thicknessso as to be compressible, deflatable and collapsible, for example of about 600 microns consisting for example of 20 layers of silicone, each having a thickness of about 30 microns, obtained in accordance with what is described above. Alternatively, said membrane 2 can be a single layer made by means of silicone moulding.

In a preferred embodiment said balloon 600 has a thickness of 0.6 mm and a diameter between about 72 and 74 mm.

On said balloon 600 an aperture (not illustrated) and three circular holes 603' (Figures 7b and 7d) are formed, identifying three substantially flat areas, each area provided with a stopper type cover 603 (Figures 7a and 7b). In a preferred embodiment said holes 603' have a diameter of about 22 mm and said stoppers 603 have an outside diameter of about 26 mm.

After said balloon 600 has been obtained, a circular conical frustum 602 (Figure 7a), hollow on the inside and made of silicone, which faces towards the outer surface 601 of said balloon 600, is glued to coincide with one of the three stoppers 603. In a preferred embodiment the conical frustum 602 has a height of about 15 mm, a base with a diameter of about 24 mm, an inner diameter of the hole of about 6 mm and a thickness of about 1 mm.

Said conical frustum 602 serves as a guide for insertion of the urethra during the surgical stage, as will be described hereunder. The other two flattened circular areas coinciding with the two stoppers 603, without the conical frustum 602, on the other hand, will serve for coupling with above mentioned respective elements in a single piece 300 as will be described hereunder.

After the silicone balloon 600 has been obtained, a first deposition of turbostratic carbon will be made on the outer surface 601, according to the prior art, after having shielded the edges of the aperture mentioned previously, then proceeding with a first vulcanization of said balloon 600.

Once the balloon 600 provided with the cone 602 with the outer surface 601 coated with turbostratic carbon like the lateral surface of the conical frustum 602 has been obtained, said balloon is turned inside out a first time through the aperture (not shown in the figure) so as to bring the surface 601 and the cone 602 from the outside to the inside of the balloon 600.

At this point the inner surface 604 opposite the surface 601 is turned to the outside: coating of said surface 604 is then performed by means of application of turbostratic carbon after suitable shielding of the surfaces 605 of the stoppers 603 now facing outwards, and of the edges of the opening for turning inside out.

A second vulcanization is subsequently carried out, after which the shielding of the surfaces 605 (Figure 7c) of the stoppers 603 is removed.

At this point it is possible to make a through hole 9 (or 9') (Figures 6 and 6a) on one or both surfaces 605 of the stoppers 603 free of the cone 602, to allow insertion and passage in said hole 9 of the tubular portion 10 of each element 300, also previously coated with turbostratic carbon, then proceeding with gluing of the surface 13 (Figure 5) on the surface 605 not coated with turbostratic carbon.

At the end of gluing each element 300 has its own conical portion 14 (Figure 5b) facing towards the outside of the balloon 600 and the tubular portion 10 facing towards the inside.

Said one-piece element 300 has been coated with turbostratic carbon both internally and externally, similarly to what was done on the balloon 600 with shielding of the surface 13, making a longitudinal cut, shielding the edges of said cut, applying the turbostratic carbon on both surfaces of the opened longitudinally cut piece 300, vulcanizing, and subsequently removing the shielding of the edges of the cut to be able to carry out gluing of said two shielded edges.

After coupling of one or two elements 300 with the balloon artificial bladder 600, the balloon 600 is turned inside out again returning thus to the situation illustrated in Figures 6 and 7a where the surface 601, the cone 602 and the tubular portion 10 of each of said elements 300 face outwards whereas the conical portion 14 of the element 300, the surface 604 of the balloon and the surface 605 of the stoppers 603 are turned towards the inside of the balloon 600.

At this point the edges of the aperture for turning the balloon inside out can be glued together in order to obtain a closed balloon 600 and thus a closed bladder.

The above mentioned gluing operations are preferably carried out with the glue 500 previously described.

The through hole 9 (or 9') is preferably made using a stainless steel neurosurgery stylet or a punch.

With the procedure thus far described an orthotopic prosthesis 1 of artificial bladder 600 coated both internally and externally with turbostratic carbon is obtained, with the conical frustum 602 and the tubular portion 10 of the one-piece element 300 faced outwards which is ready to be implanted in the patient.

During the operation the conical frustum 602 will be passed through by a stainless steel punch or neurosurgery stylet in order to pierce the stopper 603 beneath the conical frustum 602 and allow the urethra 8 to enter the artificial bladder whilst each ureter 6, 6' will be forced inside said tubular portion 10 similarly to what was described previously for the hollow element 200.

In order to give greater protection to the ureter 6, 6' inserted in said tubular portion 10 of said one-piece element 300, and a greater rigidity to said tubular portion, it is preferable also to use a sleeve 700 (Figure 8) to be applied externally to the tubular portion 10 of the element 300 as illustrated in Figure 6, once the ureter 6, 6' has been inserted.

With the process thus far described it is therefore possible to obtain another embodiment of the prosthesis 1 according to the invention where the artificial bladder 1 is a non texturized balloon 600 but is coated internally and externally with pyrolytic turbostratic carbon, which further comprises a conical frustum 602 able to connect said prosthesis 1 to said urethra 8. Furthermore, said balloon provides for the hollow element for fixing of the ureters 6. 6' to be a single piece 300 coated internally and externally with pyrolytic turbostratic carbon which is formed by a frustoconical portion 14 in addition to the widened base 13 which is connected to the tubular portion 10 which will be inserted in the through holes 9. 9', similarly to what is reported for the element 200, whereas the widened base 13 of the element 300 will come into contact with the inside surface 605 of said membrane 2, similarly to the circular base 11 of the element 200 previously described.

Numerous changes and modifications of detail within the reach of a person skilled in the art can be made to the present embodiment of the invention without thereby departing from the scope of the invention as set forth in the appended claims.

## Claims

1. An orthotopic artificial bladder prosthesis (1) comprising an enclosure or bag or balloon made from a multi-layer membrane (2) of soft and elastic silicone with a thickness so as to be compressible, deflatable or collapsible, and at least two holes (9, 9') for connecting the ureters (6, 6') to said prosthesis (1) and a further hole for passage of the urethra (8) or of a stent, the inner surface (4) of the membrane being coated with pyrolytic turbostratic carbon
**characterized in that** fixing of said ureters (6, 6°) is provided by means of a respective hollow element (200) comprising a widened flat base (11) applied inside said membrane (2) and a tubular portion (10) passing through said hole (9, 9') and inside which said ureter (6, 6') is inserted in a forced manner such as to obtain an interference fit, said tubular portion (10) being substantially perpendicular to said flat base (11) and having an inner diameter equal along its length,
and further **characterized in that** the outermost layer (3) of said multi-layer membrane (2) is coated with pyrolytic turbostratic carbon and the hollow element (200) inclusive of the tubular portion (10) is coated, both internally and externally with turbostratic pyrolytic carbon.

2. The prosthesis according to claim 1, wherein said holes (9, 9') are made in respective portions of membrane (7), said portions of membrane (7) being applied to the inner surface (4) of the membrane (2) of the prosthesis (1) to cover holes (5) previously made in said prosthesis (1).

3. The prosthesis according to claim 1, wherein a silicone glue (500) is placed on said widened base (11), said glue solidly connecting the hollow element (200) to the multi-layer membrane (2) or to the membrane (7).

4. The prosthesis according to any one of the preceding claims, wherein said tubular portion (10) has an inner diameter between 5 and 30 charrier (Ch), a thickness between 1.0 and 10 mm and a length not exceeding 5 cm.

5. The prosthesis according to any one of the preceding claims, wherein said hole (9) has a size generally between 5 and 30 Ch and is such as to permit forced passage of the tubular portion (10) of the hollow element (200).

6. The prosthesis according to any one of the preceding claims, wherein the ureter (6, 6') inserted in the tubular portion (10) of the hollow element (200) projects with respect to said widened base (11).

7. The prosthesis according to any one of the preceding claims, wherein said urethra (8) is also fixed by means of said hollow element (200).

8. The prosthesis according to claim 1 wherein said artificial bladder (1) is a non texturized balloon (600) coated internally and externally with pyrolytic turbostratic carbon, further comprising a conical frustum (602) suitable for connection of said prosthesis to said ureter (8), **characterized in that** the hollow element for fixing of said ureters (6, 6') is a single piece (300) coated internally and externally with pyrolytic turbostratic carbon and comprises a frustoconical portion (14) in addition to the widened base (13) which is connected to the tubular portion (10) passing in said hole (9, 9'), said widened base (13) being in contact with a surface (605) facing towards the inside of said membrane (2).

9. The prosthesis according to claim 8 in which said surface (605) is the bottom surface of a cover (603) placed on the outer surface (601) of said balloon (600).

10. The process for preparing the prosthesis as defined in claims 8-9 comprising
- obtaining a prosthesis in the form of a balloon (600) having an outer surface (601) and an inner surface (604), said balloon (600) consisting of a membrane (2) of soft silicone;
- obtaining on said balloon an aperture and three circular holes (603'), and closing each hole (603') with a stopper type cover (603);
- gluing onto one of the covers (603) of a circular conical frustum (602) of silicone, hollow on the inside and facing towards the outer surface (601) of said balloon (600);
- deposition of turbostratic carbon on the outer surface (601) of the balloon (600) and on the surface of said conical frustum (602), after shielding of the edges of said aperture;
- first vulcanization;
- turning inside out of said vulcanized balloon (600) through said aperture and of said surface (601) from the outside to the inside of said balloon (600) and of said surface (604) from the inside to the outside;
- coating of said surface (604) by application of turbostratic carbon after suitable shielding of the surfaces (605) of the covers (603) and of the edges of the aperture;
- second vulcanization and removal of the shielding;
- formation of a through hole (9, 9') on the surface (605) of said stopper (603) without the cone (602), insertion in said hole (9, 9') of the tubular portion (10) of the element (300), previously coated with turbostratic carbon, gluing of the widened surface (13) to the uncoated surface (605),
- subsequent turning inside out of said balloon (600) to turn said cone (602) and said tubular portion (10) of said element (300) towards the outside, and subsequent gluing together of the edges of the aperture for turning inside out.

11. The process according to claim **10** wherein the one-piece element (300) has been coated with turbostratic carbon both internally and externally, with the exception of the surface (13) by making a longitudinal cut, shielding the edges of said cut, applying turbostratic carbon to both surfaces of the opened element (300), vulcanization, and subsequently removal of the shielding of the edges of the cut, and subsequent gluing of said two shielded edges.

## Patentansprüche

1. Orthotopische künstliche Harnblasenprothese (1), die eine Umschließung oder einen Beutel oder einen Ballon, der aus einer mehrschichtigen Membran (2) aus weichem und elastischem Silikon mit einer Dicke, um kompressibel, entleerbar und verkleinerbar oder zusammenlegbar zu sein, hergestellt ist, und mindestens zwei Löcher (9, 9') zum Verbinden des Harnleiters (6, 6') mit der Prothese (1) und ein weiteres Loch für einen Durchlass der Harnröhre (8) oder eines Stents umfasst, wobei die innere Oberfläche (4) der Membran mit pyrolytischem, turbostratischem Kohlenstoff beschichtet ist,
**dadurch gekennzeichnet, dass** ein Befestigen der Harnleiter (6, 6') durch Mittel eines jeweiligen hohlen Elements (200), das eine aufgeweitete, flache Basis (11), die innerhalb der Membran (2) angewendet wird, und einen schlauchförmigen Abschnitt (10), der durch das Loch (9, 9') hindurch verläuft und in dessen Inneres der Harnleiter (6, 6') durch Druck eingeführt wird, um einen Pressverband zu erreichen, umfasst, bereitgestellt wird, wobei der schlauchförmige Abschnitt (10) im Wesentlichen zu der flachen Basis (11) senkrecht ist und einen inneren Durchmesser aufweist, der entlang seiner Länge gleich ist,
und ferner **dadurch gekennzeichnet, dass** die äußerste Schicht (3) der mehrschichtigen Membran (2) mit pyrolytischem, turbostratischem Kohlenstoff beschichtet ist und das hohle Element (200) einschließlich des schlauchförmigen Abschnitts (10) sowohl innen als auch außen mit pyrolytischem, turbostratischem Kohlenstoff beschichtet ist.

2. Prothese nach Anspruch 1, wobei die Löcher (9, 9') in entsprechenden Abschnitten einer Membran (7) hergestellt sind, wobei die Abschnitte einer Membran (7) auf die innere Oberfläche (4) der Membran (2) der Prothese (1) aufgetragen ist, um die Löcher (5), die vorher in der Prothese (1) hergestellt worden sind, zu bedecken.

3. Prothese nach Anspruch 1, wobei ein Silikonkleber (500) auf der aufgeweiteten Basis (11) angeordnet ist, wobei der Kleber das hohle Element (200) mit der mehrschichtigen Membran (2) oder mit der Membran (7) verbindet.

4. Prothese nach einem der vorhergehenden Ansprüche, wobei der schlauchförmige Abschnitt (10) einen inneren Durchmesser zwischen 5 und 30 Charrière (CH), eine Dicke zwischen 1,0 und 10 mm und eine Länge, die 5 cm nicht übersteigt, aufweist.

5. Prothese nach einem der vorhergehenden Ansprüche, wobei das Loch (9) eine Größe im Allgemeinen zwischen 5 und 30 CH aufweist und derart ist, dass es einen erzwungenen Durchlass des schlauchförmigen Abschnitts (10) des hohlen Elements (200) ermöglicht.

6. Prothese nach einem der vorhergehenden Ansprüche, wobei der Harnleiter (6, 6'), der in den schlauchförmigen Abschnitt (10) des hohlen Elements (200) eingeführt worden ist, in Bezug auf die aufgeweitete Basis (11) übersteht.

7. Prothese nach einem der vorhergehenden Ansprüche, wobei die Harnröhre (8) auch durch Mittel des hohlen Elements (200) befestigt worden ist.

8. Prothese nach Anspruch 1, wobei die künstliche Harnblase (1) ein nicht texturierter Ballon (600) ist, der intern und extern mit pyrolytischem, turbostratischem Kohlenstoff beschichtet ist, wobei sie ferner einen Kegelstumpf (602) umfasst, der für eine Verbindung der Prothese mit der Harnröhre (8) geeignet ist, **dadurch gekennzeichnet, dass** das hohle Element zum Befestigen des Harnleiters (6, 6') ein einziges Stück (300) ist, das intern und extern mit pyrolytischem, turbostratischem Kohlenstoff beschichtet ist, und dass es zusätzlich zu der aufgeweiteten Basis (13), die mit dem schlauchförmigen Abschnitt (10), der in dem Loch (9, 9') verläuft, verbunden ist, einen kegelstumpfförmigen Abschnitt (14) umfasst, wobei sich die aufgeweitete Basis (13) im Kontakt mit der Oberfläche (605) befindet, die dem Inneren der Membran (2) zugewandt ist.

9. Prothese nach Anspruch 8, in der die Oberfläche (605) die Unterseite einer Abdeckung (603) ist, die auf der äußeren Oberfläche (601) des Ballons (600) angeordnet ist.

10. Verfahren zum Herstellen der Prothese, wie sie nach den Ansprüchen 8 bis 9 definiert sind, das umfasst:
- Erreichen einer Prothese in der Form eines Ballons (600) mit einer äußeren Oberfläche (601) und einer inneren Oberfläche (604), wobei der Ballon (600) aus einer Membran (2) aus weichem Silikon besteht;
- Erreichen einer Öffnung und dreier kreisförmiger Löcher (603') auf dem Ballon, und Schließen von jedem Loch (603') mit einer Abdeckung vom Stöpseltyp (603);
- Kleben auf einer der Abdeckungen (603) eines kreisförmigen Kegelstumpfes (602) von Silikon, hohl im Innern, und der äußeren Oberfläche (601) des Ballons (600) zugewandt;
- Auftragen von turbostratischem Kohlenstoff auf die äußere Oberfläche (601) des Ballons (600) und auf die Oberfläche des Kegelstumpfes (602) nach einem Abschirmen der Kanten der Öffnung;
- erste Vulkanisierung;
- Wenden des Inneren nach außen von dem vulkanisierten Ballon (600) durch die Öffnung und Wenden von der Oberfläche (601) von der Außenseite nach dem Inneren des Ballons (600) und Wenden von der Oberfläche (604) von dem Inneren nach außen;
- Beschichten der Oberfläche (604) durch Auftragung von turbostratischem Kohlenstoff nach geeigneter Abschirmung der Oberflächen (605) der Abdeckungen (603) und der Kanten der Öffnung;
- zweite Vulkanisierung und Entfernung der Abschirmung;
- Bilden eines Durchgangslochs (9, 9') auf der Oberflache (605) des Stöpsels (603) ohne den Kegelstumpf (602), Einsetzung in das Loch (9, 9') des schlauchförmigen Abschnitts (10) des Elements (300), vorher beschichtet mit turbostratischem Kohlenstoff, Kleben der aufgeweiteten Oberfläche (13) auf die unbeschichtete Oberfläche (605),
- darauffolgend Wenden des Inneren nach außen von dem Ballon (600), um den Kegelstumpf (602) und den schlauchförmigen Abschnitt (10) des Elements (300) nach außen zu wenden, und darauffolgend Zusammenkleben der Kanten der Öffnung, um das Innere nach außen zu wenden.

11. Prozess nach Anspruch 10, wobei das Einstück-Element (300) mit der Ausnahme der Oberfläche (13) sowohl innen als auch außen mit pyrolytischem, turbostratischem Kohlenstoff beschichtet worden ist durch Herstellen eines Längsschnitts, Abschirmen der Kanten des Schnitts, Auftragen des turbostratischen Kohlenstoffs auf beide Oberflächen des geöffneten Elements (300), Vulkanisation und nachfolgende Entfernung der Abschirmung der Kanten des Schnitts und nachfolgendes Kleben der zwei abgeschirmten Kanten.

## Revendications

1. Prothèse pour vessie artificielle orthotopique (1) comprenant une enceinte, un sac ou un ballon fabriqué à partir d'une membrane multicouches (2) de silicone souple et élastique avec une épaisseur de façon à être compressible, dégonflable ou pliable, au moins deux trous (9, 9') pour relier les uretères (6, 6') de ladite prothèse (1) et un autre trou pour le passage de l'urètre (8) ou d'un stent, la surface intérieure (4) de la membrane étant revêtue avec du carbone turbostratique par pyrolyse,
**caractérisée en ce que** la fixation desdites uretères (6, 6') est obtenue par l'intermédiaire d'un élément creux respectif (200) comprenant une base plate élargie (11) appliquée à l'intérieur de ladite membrane (2) et une partie tubulaire (10) passant à travers ledit trou (9, 9') et à l'intérieur duquel ledit uretère (6, 6') est inséré d'une manière forcée de façon à obtenir une interférence, ladite partie tubulaire (10) étant sensiblement perpendiculaire à ladite base plate (11) et ayant une diamètre intérieur égal sur toute sa longueur,
et **caractérisée en outre en ce que** la couche extérieure (3) de ladite membrane multicouches (2) est revêtue de carbone turbostratique par pyrolyse et l'élément creux (200) de la partie tubulaire (10) est revêtu, à l'intérieur et à l'extérieur, de carbone turbostratique par pyrolyse.

2. Prothèse selon la revendication 1, où lesdits trous (9, 9') sont créés dans des parties respectives de la membrane (7), lesdites parties respectives de la membrane (7) étant appliquées à la surface interne (4) de la membrane (2) de la prothèse (1) pour couvrir les trous (5) précédemment créés dans ladite prothèse (1).

3. Prothèse selon la revendication 1, où une colle à base de silicone (500) est placée sur ladite base élargie (11), ladite colle reliant solidement l'élément creux (200) à la membrane multicouches (2) ou à la membrane (7).

4. Prothèse selon l'une quelconque des revendications précédentes, où ladite partie tubulaire (10) a un diamètre interne entre 5 et 30 French (Fr), une épaisseur allant de 1,0 à 10 mm et une longueur de 5 cm maximum.

5. Prothèse selon l'une quelconque des revendications précédentes, où ledit trou (9) a une taille située généralement entre 5 et 30 Fr pour permettre un passage forcé de la partie tubulaire (10) de l'élément creux (200).

6. Prothèse selon l'une quelconque des revendications précédentes, où l'uretère (6, 6') inséré dans la partie tubulaire (10) de l'élément creux (200) se projette par rapport à la base élargie (11).

7. Prothèse selon l'une quelconque des revendications précédentes, où ledit urètre (8) est également fixé grâce audit élément creux (200).

8. Prothèse selon la revendication 1, dans laquelle ladite vessie artificielle (1) est un ballon non texturée (600) revêtue à l'intérieur et à l'extérieur avec du carbone turbostratique par pyrolyse, comprenant en outre un tronc conique (602) adapté pour relier ladite prothèse à ladite uretère (8), **caractérisée en ce que** l'élément creux pour fixer lesdites uretères (6, 6') est d'un seul bloc (300) revêtue à l'intérieur et à l'extérieur avec du carbone turbostratique par pyrolyse et comprend une partie tronconique (14), en plus de la base élargie (13), relié à la partie tubulaire (10) passant dans ledit creux (9, 9'), ladite base élargie (13) étant en contact avec une surface (605) faisant face à l'intérieur de ladite membrane (2).

9. Prothèse selon la revendication 8 où ladite surface (605) est la surface inférieure d'un couvercle (603) placé sur la surface extérieure (601) dudit ballon (600).

10. Procédé de préparation de la prothèse telle que définie dans les revendications 8-9, comprenant :
- l'obtention d'une prothèse sous la forme d'un ballon (600) ayant une surface extérieure (601) et une surface intérieure (604), ledit ballonnet (600) étant constitué d'une membrane (2) de silicone souple ;
- l'obtention sur ledit ballon d'une ouverture et de trois trous circulaires (603'), et la fermeture de chaque trou (603') avec un couvercle de type bouchon (603) ;
- le collage sur l'un des couvercles (603) d'un tronc conique circulaire (602) en silicone, creux à l'intérieur et tournée vers la surface extérieure (601) dudit ballon (600) ;
- le dépôt de carbone turbostratique sur la surface extérieure (601) du ballon (600) et sur la surface dudit tronc conique (602), après protection des bords de ladite ouverture ;
- la première vulcanisation ;
- le retournement de l'intérieur dudit ballon vulcanisé (600) à travers ladite ouverture et de ladite surface (601) de l'extérieur vers l'intérieur dudit ballon (600) et de ladite surface (604) de l'intérieur vers l'extérieur ;
- le revêtement de ladite surface (604) par application de carbone turbostratique après protection convenable des surfaces (605) des couvercles (603) et des bords de l'ouverture ;
- la deuxième vulcanisation et l'élimination de la protection ;
- la formation d'un trou (9, 9') à travers la surface (605) dudit bouchon (603) sans le cône (602), l'insertion dans ledit trou (9, 9') de la partie tubulaire (10) de l'élément (300), préalablement revêtu de carbone turbostratique, pour coller la surface élargie (13) à la surface non revêtue (605),
- le retournement ultérieur de l'intérieur dudit ballon (600) pour tourner ledit cône (602) et ladite partie tubulaire (10) dudit élément (300) vers l'extérieur, et le collage ultérieur des bords de l'ouverture pour le tourner à l'envers.

11. Procédé selon la revendication 10, dans lequel l'élément d'un seul bloc (300) a été revêtu avec du carbone turbostratique à l'intérieur et à l'extérieur, à l'exception de la surface (13), en effectuant une coupe longitudinale, en protégeant les bords de ladite coupure, en appliquant du carbone turbostratique aux deux surfaces de l'élément ouvert (300), en vulcanisant, et par la suite en éliminant la protection des bords de la coupure et en collant ensuite lesdits deux bords protégés.
